# EUROPEAN PATENT APPLICATION

(11) **EP 4 450 522 A2**
(43) Date of publication of application: **23.10.2024**
(21) Application number: 23731492.7
(22) Date of filing: 03.02.2023
(51) Int. Cl.: C07K 16/28, C12N 15/13, A61K 47/68

(54) **ANTIBODY AGAINST OX40 AND MEDICAL USE THEREOF**

(30) Priority: 14.12.2021 CN 202111525230
(71) Applicant: INNOLAKE BIOPHARMA (HANGZHOU) CO., LTD, Zhejiang 310018 (CN)
(72) Inventor: QIU, Junzhuan, Hangzhou, Zhejiang 310018 (CN); LI, Zhongliang, Hangzhou, Zhejiang 310018 (CN); SUN, Kai, Hangzhou, Zhejiang 310018 (CN); CHEN, Junyong, Hangzhou, Zhejiang 310018 (CN); SUN, Jian, Hangzhou, Zhejiang 310018 (CN); WANG, Zhensheng, Hangzhou, Zhejiang 310018 (CN); CHEN, Rulei, Zhejiang, 310018 (CN); XIA, Mingde, Zhejiang, 310018 (CN)
(74) Representative: Laufhütte, Dieter
(86) International application number: PCT/CN2023/074425
(87) International publication number: WO 2023/109976

(57) **Abstract**

The present invention relates to the technical field of immunotherapy and molecular immunology, in particular to an antibody against OX40 and the medical use. Provided in the present invention is a specific antibody or an antigen binding fragment thereof, which has a high affinity to OX40 and can block the interaction and signaling of OX40/OX40L.

## Description

### CROSS-REFERENCE TO RELATED APPLICATIONS

This application claims priority to Chinese patent application No. 202111525230.6, filed with the China National Intellectual Property Administration on December 14, 2021 and entitled "ANTIBODY AGAINST OX40 AND MEDICAL USE THEREOF", which is incorporated herein by reference in its entirety.

### TECHNICAL FIELD

The present disclosure relates to the field of immunotherapy and molecular immunology, and more particularly, to an antibody against OX40 and medical use thereof.

### BACKGROUND

OX40 (also known as CD134, ACT35, or TNFRSF4) is a member of the TNFR superfamily, and is a membrane glycoprotein with a molecular weight of 50 kD consisting of a cytoplasmic tail region, a transmembrane region, and an extracellular region. OX40 is substantially not expressed in non-activated naive T cells, but is highly expressed in activated T cells (Paterson, et al., (1987), Mol. Immunol. 24:1281-90; Mallett, et al., (1990), EMBO J. 9:1063-8; Calderhead, et al., (1993), J. Immunol. 151:5261-71). OX40 ligand (OX40L) is a 34 kD type II membrane protein expressed as a trimer on antigen-presenting cells (Stuber, et al., (1995), Immunity 2:507-21; Ohshima, et al., (1997), J. Immunol. 159:3838-48; Al-Shamkhani, et al., (1997), J. Biol. Chem. 272: 5275-5282). After antigen stimulation, OX40 gene-knockout mice showed reduced CD4 and CD8 T cell proliferation and defective immunological memory responses, indicating that endogenous OX40 is crucial in T cell immune regulation (Kopf, et al., (1999), Immunity 11:699-708; Redmond, et al., (2009), Crit. Rev. Immunol. 29:187-201; Croft, et al., (2010), Annu. Rev. Immunol. 28:57-78).

OX40 is essential for T cell proliferation and survival, and T cells lacking OX40 fail to survive effectively during and after division, which is associated with decreased expression levels of anti-apoptotic Bcl-2 family members (Rogers, et al., (2001), Immunity 15:445-455; Gramaglia, et al., (2000), J. Immunol. 165:3043-3050). The binding of natural ligands (OX40L) or activating antibodies to OX40 receptors on the surface of T cells can activate multiple signaling pathways, including transcription factor pathways, namely nuclear factor kappa light chain enhancer of activated B (NF-kB) and activated T cell transcription factor (NFAT), thereby increasing the expression of multiple protein molecules such as survivin, cyclin A, cyclin-dependent kinases and Bcl-2 anti-apoptotic molecules, and increasing the survival and cell proliferation of multiple effector T cell subsets after antigen stimulation (Rogers, et al., (2001), Immunity. 15:445-455; Song, et al, (2005), Immunity. 22: 621-31; Sun, et al., (2018), Cell Death & Disease 9:616-628; Croft, et al., (2010), Annu. Rev. Immunol. 28:57-78). OX40L-OX40 signaling can regulate cytokine secretion in T cells, antigen-presenting cells, and NKT cells, and regulate signal transduction in cytokine receptor pathways (Gramaglia, et al., (1998), J. Immunol. 161 :6510-17; Diana, et al., (2009), Immunity. 30:289-99; Ohshima, et al., (1997), J. Immunol. 159:3838-48). OX40 is mainly expressed in activated CD4+T cells and some activated CD8+T cells (Salek-Ardakani S etc. (2006) Curr. Immunol. Rev. 2: 37-53), is highly expressed in memory T cells associated with some chronic inflammatory and autoimmune diseases, and has a ligand (OX40L) that is expressed in presenting cells along with cell activation.

In recent years, various activating anti-OX40 antibodies have been subjected to clinical trials for cancer immunotherapy, and inhibitory OX40 antibodies have shown protective effects in animal models of various autoimmune diseases such as asthma, irritable bowel disease, transplant rejection, autoimmune diabetes, graft-versus-host disease (GvHD), experimental autoimmune encephalomyelitis, and atopic dermatitis (AD). Among them, atopic dermatitis is a chronic recurrent inflammatory skin disease characterized by severe itching (e.g. pruritus) and scaly and dry eczematous lesions, which have a significant impact on patients' quality of life. Currently, there are still limited therapeutic options for AD, and blocking the OX40/OX40L signaling pathway can specifically inhibit a function of disease-inducing effector T cells without causing widespread immunosuppression.

### SUMMARY

A first objective of the present disclosure is to provide an antibody capable of specifically recognizing OX40 or an antigen-binding fragment thereof, including heavy chain complementarity-determining regions HCDR1, HCDR2, and HCDR3, and light chain complementarity-determining regions LCDR1, LCDR2, and LCDR3. The amino acid sequences of HCDR1, HCDR2, HCDR3, LCDR1, LCDR2, and LCDR3 are selected from one of complementarity-determining region combinations designated as a to j:

| Combination name | HCDR1 | HCDR2 | HCDR3 | LCDR1 | LCDR2 | LCDR3 |
|---|---|---|---|---|---|---|
| a | SEQ ID NO: 13 | SEQ ID NO: 14 | SEQ ID NO: 15 | SEQ ID NO: 16 | SEQ ID NO: 17 | SEQ ID NO: 18 |
| b | SEQ ID NO: 19 | SEQ ID NO: 20 | SEQ ID NO: 21 | SEQ ID NO: 22 | SEQ ID NO: 23 | SEQ ID NO: 24 |
| c | SEQ ID NO: 25 | SEQ ID NO: 8 | SEQ ID NO: 9 | SEQ ID NO: 37 | SEQ ID NO: 38 | SEQ ID NO: 39 |
| d | SEQ ID NO: 10 | SEQ ID NO: 11 | SEQ ID NO: 12 | SEQ ID NO: 40 | SEQ ID NO: 41 | SEQ ID NO: 42 |
| e | SEQ ID NO: 13 | SEQ ID NO: 14 | SEQ ID NO: 15 | SEQ ID NO: 43 | SEQ ID NO: 44 | SEQ ID NO: 45 |
| f | SEQ ID NO: 16 | SEQ ID NO: 17 | SEQ ID NO: 18 | SEQ ID NO: 46 | SEQ ID NO: 47 | SEQ ID NO: 48 |

A second objective of the present disclosure is to provide an isolated nucleic acid encoding the antibody or antigen-binding fragment thereof.

A third objective of the present disclosure is to provide a vector including the nucleic acid.

A fourth objective of the present disclosure is to provide a host cell including the nucleic acid or the vector.

A fifth objective of the present disclosure is to provide a method for producing an antibody or an antigen-binding fragment thereof, including:
culturing the host cell as described above under suitable culture conditions; and
recovering an antibody or antigen-binding fragment thereof thus produced from the culture medium or the cultured cells.

A sixth objective of the present disclosure is to provide a conjugate including the antibody or antigen-binding fragment thereof and a conjugate moiety, where the conjugate moiety is selected from one or more of a radionuclide, a pharmaceutical, a toxin, a cytokine, a cytokine receptor fragment, an enzyme, fluorescein, and biotin.

A seventh objective of the present disclosure is to provide a composition or a combination product including one or more of the antibody or antigen-binding fragment thereof; and/or the conjugate.

An eighth objective of the present disclosure is to provide use of the antibody or antigen-binding fragment thereof, and/or the conjugate in preparation of a medicament, where the medicament is for treating one or more of tumors, asthma, irritable bowel disease, transplant rejection, autoimmune diabetes, graft-versus-host disease, experimental autoimmune encephalomyelitis, and atopic dermatitis.

The present disclosure provides specific antibodies or antigen-binding fragments thereof with high affinity for OX40, capable of blocking interaction and signal transduction of OX40/OX40L. These antibodies or antigen-binding fragments thereof can be used for inhibition and regulation of the function and level of OX40 and significant inhibition of the immune inflammatory response of the body, and have broad application prospects in the development of related drugs for the treatment of chronic inflammatory or autoimmune diseases.

### BRIEF DESCRIPTION OF DRAWINGS

In order to more clearly describe the technical solutions in the specific embodiments of the present disclosure or in the prior art, the accompanying drawings necessary to describe the specific embodiments or the prior art are briefly described below. Obviously, the accompanying drawings in the following description show only some embodiments of the present disclosure, and those skilled in the art can still derive other drawings from these accompanying drawings without creative effort. In addition, the conclusions given in the drawings below are the test data provided by the methods of the corresponding specific embodiments of the present disclosure.
FIG. 1 shows an ELSIA assay of the EC50 of OX40 hybridoma antibody bound to OX40-hFc protein;
FIG. 2 shows a FACS assay of the EC50 of OX40 hybridoma antibody bound to 293T-OX40 cell;
FIG. 3 shows a reporter gene assay of the IC50 of OX40 hybridoma antibody to block activation of OX40L induced signal;
FIG. 4 shows a reporter gene assay of the IC50 of OX40 chimeric antibody blocking OX40L-induced signaling pathway, where A represents WT antibody and B represents DE mutant antibody;
FIG. 5 shows a FACS assay of the inhibitory effect of OX40 chimeric antibody on CD4+T cell proliferation in MLR, where A represents WT antibody and B represents DE mutant antibody;
FIG. 6 shows a FACS assay of the inhibitory effect of OX40 chimeric antibody on CD8+T cell proliferation induced by CEF, where A represents WT antibody and B represents DE mutant antibody;
FIG. 7 shows a FACS assay of EC50 of OX40 humanized antibody bound to 293T-OX40 cell;
FIG. 8 shows a reporter gene assay of IC50 of OX40 humanized antibody blocking OX40L-induced signaling pathway;
FIG. 9 shows a FACS assay of the inhibitory effect of OX40 humanized antibody on CD4+T cell proliferation in MLR, where A represents Donor1, and B represents Donor2;
FIG. 10 shows a FACS assay of the inhibitory effect of OX40 humanized antibody on CD8+T cell proliferation induced by CEF, where A represents Donor1, and B represents Donor2; and
FIG. 11 shows an LDH assay of the ADCC response induced by OX40 humanized antibody.

### DETAILED DESCRIPTION

Reference is made in detail to embodiments of the present disclosure, and one or more examples are described below. Each example is provided to illustrate rather than limit the present disclosure. In practice, apparently, those skilled in the art can make various modifications and changes to the present disclosure without departing from the scope or spirit of the present disclosure. For example, features illustrated or described as a part of one embodiment can be used in another embodiment to create yet another embodiment.

Unless otherwise specified, all terms (including technical and scientific terms) used to disclose the present disclosure have the same meanings as commonly understood by those ordinary skills in the art to which the present disclosure belongs. With reference to further guidance, the following definitions are used to better understand the instructions in the present disclosure. The terms used herein in the description of the present disclosure are for the purpose of describing specific embodiments only, and are not intended to limit the present disclosure.

A selection range of a term "and/or" used herein includes any one of two or more related listed items, and a combination of any one or all of the related listed items, and the combination of any one or all of the related listed items includes a combination of any two or more related listed items or all of the related listed items. It should be noted that where at least three items are linked "and/or", it should be understood that, in this application, technical solutions undoubtedly include technical solutions logically linked by "and", and also undoubtedly include technical solutions logically linked by "or". For example, "A and/or B" includes three parallel solutions: A, B, and both A and B. For another example, technical solutions of "A, B, C and/or D" include any one of A, B, C or D (namely, a technical solution logically linked by "or"), and also include a combination of any one and all of A, B, C, and D, that is, a combination of any two or three of A, B, C, and D and a combination of four of A, B, C, and D (namely, technical solutions logically linked by "or").

The terms "comprising", "including", and "containing" used in the present disclosure are synonymous, are inclusive or open-ended, and do not exclude additional uncited members, elements, or method steps.

The numerical ranges in the present disclosure defined by endpoints include all values, fractions, and the cited endpoints subsumed within the range.

In the present disclosure, descriptions such as "a plurality of" and "multiple" refer to two or more than two unless otherwise specified.

In the present disclosure, technical features in open-ended descriptions include closed-ended technical solutions including the listed features, and also include open-ended technical solutions including the listed features.

In the present disclosure, "preferred", "better", "preferable" and "proper" are only intended to describe embodiments or examples with better effects, and should not be construed as limiting the protection scope of the present disclosure. In the present disclosure, the terms "optionally," "optional," and "option" mean that they may or may not be present, i.e., selected from two coexisting schemes "present" or "absent." If there are multiple "options" in a technical solution, each "option" is independent, unless otherwise specified, if there is no contradiction or mutual constraint.

All documents referenced in the present disclosure are cited by reference in this application as if each document was individually cited by reference. Unless in conflict with the inventive purpose and/or technical solution of this application, the cited references involved in the present disclosure are cited in their entirety and for all purposes. When the present disclosure involves the reference, definitions of a relevant technical feature, term, noun, phrase, and the like in the reference are also cited. When the present disclosure refers to the reference, examples and preferred methods of the cited relevant technical characteristic may also be incorporated into this application by reference, provided that the present disclosure can be achieved. It should be understood that if the cited content conflicts with the description in this application, this application shall prevail or be amended adaptively based on the description of this application.

The present disclosure relates to an antibody capable of specifically recognizing OX40 or an antigen-binding fragment thereof, including heavy chain complementarity-determining regions HCDR1, HCDR2, and HCDR3, and light chain complementarity-determining regions LCDR1, LCDR2, and LCDR3, where the amino acid sequences of HCDR1, HCDR2, HCDR3, LCDR1, LCDR2, and LCDR3 are selected from one of complementarity-determining region combinations designated as a to j:

| Combination name | HCDR1 | HCDR2 | HCDR3 | LCDR1 | LCDR2 | LCDR3 |
|---|---|---|---|---|---|---|
| a | SEQ ID NO: 13 | SEQ ID NO: 14 | SEQ ID NO: 15 | SEQ ID NO: 16 | SEQ ID NO: 17 | SEQ ID NO: 18 |
| b | SEQ ID NO: 19 | SEQ ID NO: 20 | SEQ ID NO: 21 | SEQ ID NO: 22 | SEQ ID NO: 23 | SEQ ID NO: 24 |
| c | SEQ ID NO: 25 | SEQ ID NO: 8 | SEQ ID NO: 9 | SEQ ID NO: 37 | SEQ ID NO: 38 | SEQ ID NO: 39 |
| d | SEQ ID NO: 10 | SEQ ID NO: 11 | SEQ ID NO: 12 | SEQ ID NO: 40 | SEQ ID NO: 41 | SEQ ID NO: 42 |
| e | SEQ ID NO: 13 | SEQ ID NO: 14 | SEQ ID NO: 15 | SEQ ID NO: 43 | SEQ ID NO: 44 | SEQ ID NO: 45 |
| f | SEQ ID NO: 16 | SEQ ID NO: 17 | SEQ ID NO: 18 | SEQ ID NO: 46 | SEQ ID NO: 47 | SEQ ID NO: 48 |

In some embodiments, the antibody or the antigen-binding fragment thereof includes a variable region, where the a heavy chain variable region (HCVR) and a light chain variable region (LCVR) of the variable region have a sequence combination selected from one of variable region sequence combinations designated as A to F:

| Combination name | HCVR | LCVR |
|---|---|---|
| A | SEQ ID NO: 1 | SEQ ID NO: 2 |
| B | SEQ ID NO: 3 | SEQ ID NO: 4 |
| C | SEQ ID NO: 5 | SEQ ID NO: 6 |
| D | SEQ ID NO: 7 | SEQ ID NO: 8 |
| E | SEQ ID NO: 9 | SEQ ID NO: 10 |
| F | SEQ ID NO: 11 | SEQ ID NO: 12 |

In some embodiments, the complementarity-determining region combination is selected from one of the complementarity-determining region combinations designated as b, c, and d, and the antibody or the antigen-binding fragment thereof has a humanized variable region.

The term "humanized variable region", also known as a CDR-grafted antibody variable region, refers to an antibody produced by transplanting the first animal-derived CDR sequence into human antibody variable region frameworks, that is, different types of human germ line antibody framework sequences. The humanized antibody can overcome a heterologous reaction induced by chimeric antibodies due to carrying a large amount of murine protein components. Such framework sequences can be obtained from public DNA databases or published references containing germline antibody gene sequences. For example, germline DNA sequences of the human heavy and light chain variable region genes are available in the "VBase" human germline sequence database (www.mrccpe.com.ac.uk/vbase) and can be found in Sequences of Proteins of Immunological Interest (Kabat, E.A. et al., 1991, 5th ed.) To avoid both a decrease in immunogenicity and a consequent decrease in activity, a minimum of reverse mutation or back mutation can be performed on the human antibody variable region framework sequence to maintain activity. The humanized antibody in the present disclosure also includes a mature humanized antibody obtained after the phage has shown affinity for the CDR.

In some embodiments, the humanized heavy chain variable region (HCVR) and light chain variable region (LCVR) combinations of respective complementarity-determining region combinations designated as b, c, and d are designated as B', C' and D', respectively:

| Combination name | HCVR | LCVR |
|---|---|---|
| B' | SEQ ID NO: 49 | SEQ ID NO: 50 |
| C' | SEQ ID NO: 51 | SEQ ID NO: 52 |
| D' | SEQ ID NO: 53 | SEQ ID NO: 54 |

Variants of the antibody or the antigen-binding fragment thereof are also within the scope of the present disclosure. The antibody or the antigen-binding fragment thereof in the present disclosure includes a heavy chain complementarity-determining region HCDR1, HCDR2, and HCDR3 and a light chain complementarity-determining region LCDR1, LCDR2, and LCDR3. Compared with any one of the complementarity-determining region combinations designated as a to f, the sequences of HCDR1, HCDR2, HCDR3, LCDR1, LCDR2, and LCDR3 each contain mutations of up to 3 amino acids (e.g. substitution, deletion or addition of 1, 2 or 3 amino acids or any combination thereof). Preferably, the mutations are conservative mutations. In some embodiments, compared to any one of the complementarity-determining region combinations designated as a to f, the sequences of HCDR1, HCDR2, HCDR3, LCDR1, LCDR2, and LCDR3 of the antibody or the antigen-binding fragment thereof each contain a substitution of up to 3 amino acids (e.g. a substitution of 1, 2 or 3 amino acids).

In some embodiments, the antibody or the antigen-binding fragment thereof in the present disclosure includes a variable region, and an amino acid sequence of the variable region includes a sequence having at least 80%, 85%, 90%, 91%, 92%, 93%, 94%, 95%, 96%, 97%, 98% or 99% identity to any one or more of the sequences of SEQ ID NOs: 1 to 12 and SEQ ID NOs: 49 to 54. In some embodiments, the antibody or the antigen-binding fragment thereof includes HCVR and LCVR, and an amino acid sequence of HCVR includes a sequence having at least 80%, 85%, 90%, 91%, 92%, 93%, 94%, 95%, 96%, 97%, 98% or 99% identity to any one of the sequences of SEQ ID NOs: 1, 3, 5, 7, 9, 11, 49, 51 and 53; and/or an amino acid sequence of LCVR including a sequence having at least 80%, 85%, 90%, 91%, 92%, 93%, 94%, 95%, 96%, 97%, 98% or 99% identity to any one of the sequences of SEQ ID NOs: 2, 4, 6, 8, 10, 12, 50, 52 and 54.

In some cases, the variant is obtained by conservative mutation (e.g. conservative substitution or modification) in the antibody sequence provided in the present disclosure. "Conservative mutation" refers to a mutation that can normally maintain a function of a protein, and is preferably a conservative substitution. "Conservative substitution" refers to a substitution of an amino acid in the protein with another amino acid having similar characteristics (e.g. charge, side chain size, hydrophobicity/hydrophilicity, backbone conformation, and rigidity), allowing frequent changes without altering the biological activity of the protein.

Substitutions generally regarded as conservative substitutions include substitutions among aliphatic amino acids Ala, Val, Leu, and Ile, exchange of hydroxyl residues Ser and Thr, exchange of acidic residues Asp and Glu, exchange of amide residues Asn and Gln, exchange of basic residues Lys and Arg, and substitution between aromatic residues Phe and Tyr. Those skilled in the art know that the substitution of a single amino acid in a non-essential region of a peptide does not typically substantially change the biological activity (Watson et al. (1987), Molecular Biology of the Gene, The Benjamin/Cummings Pub. Co., p. 224, (4th ed.)). In addition, substitution with a structurally or functionally similar amino acid is unlikely to disrupt biological activity.

Modifications may be performed to obtain a derivative by a natural process (e.g. processing and other post-translational modifications) or by a chemical modification technique. For example, chemical modification is performed by adding one or more polyethylene glycol molecules, sugar, phosphate, and/or other similar molecules, where one or more molecules are not naturally attached to the protein. Derivatives include salts. Such chemical modifications are described in detail in basic textbooks and more specific monographs, as well as in a large number of research papers, and are well known to those skilled in the art. It should be understood that the same type of modification may be present at several sites in the given antibody or antigen-binding fragment thereof to the same or different extent. In addition, a given antibody or antigen-binding fragment thereof may include many types of modifications. The modification may occur at any site in the antibody or the antigen-binding fragment thereof, including a peptide backbone, an amino acid side chain, and an amino or carboxy terminus. The modification includes, for example, acetylation, acylation, ADP-ribosylation, amidation, covalent ligation to flavin, covalent ligation to a heme moiety, covalent ligation to a nucleotide or a nucleotide derivative, covalent ligation to a lipid or a lipid derivative, covalent ligation to phosphatidylinositol, crosslinking, cyclization, formation of a disulfide bond, demethylation, formation of covalent crosslink, formation of cysteine, formation of pyroglutamic acid, methylation, γ-carboxylation, glycosylation, formation of GPI anchor, hydroxylation, iodination, methylation, myristoylation, oxidation, proteolytic processing, phosphorylation, isoprenylation, racemization, glycosylation, lipid ligation, sulfation, γ-carboxylation of a glutamic acid residue, alkylation, ADP-ribosylation, selenization, sulfation, transfer RNA-mediated addition of an amino acid to a protein, (e.g. arginine acylation), and ubiquitination. The modification may also involve binding to vitamins such as biotin, folic acid or vitamin B12. See for example, ProteinsStructure And Molecular Properties, 2nd Ed., T. E. Creighton, W. H. Freeman and Company, New York (1993) & Wold, F., "Posttranslational Protein Modifications: Perspectives and Prospects", pgs. 1-12 in Posttranslational Covalent Modification Of Proteins, B. C. Johnson, Ed., Academic Press, New York (1983); Seifter et al., MetH. Enzymol. 182: 626-646 (1990) & Rattan et al., "Protein Synthesis: Posttranslational Modifications and Aging", Ann. N.Y Acad. Sci. 663: 48-62 1992.

The variant retains the ability to specifically bind OX40. Those skilled in the art can determine a suitable variant of the antigen-binding molecule illustrated herein by using well-known technologies. In certain embodiments, those skilled in the art can identify regions in the antibody or antigen-binding fragment thereof that are unimportant for the activity of specifically binding OX40 and modify suitable regions without destroying the activity.

In the present disclosure, the technical term "antibody" is a protein for binding to a specific antigen, and generally refers to all proteins and protein fragments including CDR regions, in particular full-length antibodies. The term "full-length antibody" includes polyclonal and monoclonal antibodies, and the term "antigen-binding fragment" is a substance including part or all of the CDRs of an antibody and lacking at least some of the amino acids present in a full-length chain, but still capable of specifically binding to the antigen. Such a fragment is biologically active because the fragment binds to a target antigen and can compete with another antigen-binding molecule (including an intact antibody) to bind a given epitope. In some embodiments, the antibody capable of specifically recognizing OX40 or an antigen-binding fragment thereof, or a variant thereof, has the function of blocking the binding of OX40 to its receptor OX40L. In one aspect, such a fragment includes a single heavy chain and a single light chain, or a part thereof. The fragment may be generated by recombinant nucleic acid technology or by enzymolysis or chemical lysis of an antigen-binding molecule (including an intact antibody).

The term "complementarity-determining region" or "CDR" refers to a hypervariable region of the heavy and light chains of an immunoglobulin, as defined by Kabat et al. (Kabat et al., Sequences of proteins of immunological interest, 5th Ed" US Department of Health and Human Services, NIH, 1991, and later editions) in the present disclosure. There are three heavy chain CDRs (HCDRs) and three light chain CDRs (LCDRs). As used herein, the terms "CDR" and "CDRs" refer to a region including one or more or even all of the major amino acid residues that promote the binding affinity between the antibody and the antigen or the epitope recognized by the antibody, depending on the specific context.

The term "antigen-binding fragment" includes antigen compound binding fragments of these antibodies, including Fab, F(ab')₂, Fd, Fv, scFv, and minimal recognition units of the antibodies, and single chain derivatives of these antibodies and fragments, such as scFv-Fc, preferably, F(ab')₂, Fab and scFv.

The terms "specific binding" and "specifically binding" refer to the binding of an antibody to an epitope on a predetermined antigen. Typically, the antibody binds at affinity (K_{D}) of approximately less than 10⁻⁷M, for example, approximately less than 10⁻⁸ M, 10⁻⁹ M, 10⁻¹⁰ M, 10⁻¹¹ M, or 10⁻¹² M, or stronger affinity.

In some embodiments, the antibody or the antigen-binding fragment thereof also includes an antibody constant region sequence.

In some embodiments, the antibody has a constant region, and a heavy chain constant region sequence is selected from any constant region sequence of IgG, IgA, IgM, IgE, and IgD.

In some embodiments, a light chain constant region is a κ or λ chain.

In some embodiments, a species origin of the heavy chain constant region and/or the light chain constant region is selected from rat, mouse, rabbit, cattle, horse, pig, sheep, dog, cat, camel, donkey, deer, marten, chicken, duck, goose, or human.

In some embodiments, the heavy chain constant region is human IgG, and is further divided into a subclass, for example, any of IgG1, IgG2, IgG3 or IgG4. In some embodiments, Ser239, Ala330, and Ile332 of IgG1 are mutated to Asp, Leu, and Glu respectively.

The present disclosure further relates to a nucleic acid encoding the antibody or antigen-binding fragment thereof.

Herein, nucleic acids include variants thereof with conservative substitutions (e.g., degenerate codon substitutions) and complementary sequences, and also include variants optimized for higher expression efficiency in desired host cells through codon optimization. The nucleic acid is typically RNA or DNA and includes a gene, a cDNA molecule, an mRNA molecule, and their fragments, such as an oligonucleotide. The nucleic acid molecule may be single-stranded or double-stranded but is preferably double-stranded DNA. When forming a functional relationship with another nucleic acid sequence, the nucleic acid is "effectively connected". For example, if a promoter or enhancer affects the transcription of a coding sequence, then the promoter or enhancer is effectively connected to the coding sequence. When incorporated into a vector, the nucleic acid molecule is preferably DNA nucleic acid.

In addition, since the antibody is a membrane protein, the nucleic acid typically carries a signal peptide sequence.

The present disclosure further relates to a vector including the nucleic acid.

The term "vector" refers to a nucleic acid delivery vehicle into which a polynucleotide can be inserted. When a vector enables expression of an inserted polynucleotide-encoded protein, the vector is referred to as an expression vector. The vector may be introduced into a host cell via transformation, transduction, or transfection, so that an element of a genetic material carried by the vector is expressed in the host cell. The vector is well-known to those skilled in the art and includes, but is not limited to, a plasmid, a phagemid, a cosmid, an artificial chromosome (e.g. a yeast artificial chromosome (YAC), a bacterial artificial chromosome (BAC), or a P1 derived artificial chromosome (PAC)), a phage (λ phage or M13 phage), and an animal virus. An animal virus that can serve as the vector includes, but is not limited to, retrovirus (including lentivirus), adenovirus, adeno-associated virus, herpesvirus (e.g. herpes simplex virus), poxvirus, baculovirus, papillomavirus, and papillomavirus (e.g. SV40). In some embodiments, the vector in the present disclosure includes a regulatory element commonly used in genetic engineering, for example, the enhancer, the promoter, an internal ribosome entry site (IRES) and another expression control element (e.g. a transcription termination signal, or a polyadenylation signal and a polyU sequence).

In the present disclosure, the vector may be a composition, for example, a mixture of multiple plasmids, where different plasmids carry parts of the antibody or the antigen-binding fragment.

The present disclosure further provides a host cell including the nucleic acid or the vector.

Host cells or cell lines suitable for expressing the antigen-binding protein in the present disclosure include: mammalian cells such as NS0, Sp2/0, CHO, COS, HEK, fibroblasts and myeloma cells. Human cells may be used to allow modification of the molecule in a human glycosylation pattern. Alternatively, other eukaryotic cell lines may be used. Methods for the selection of suitable mammalian host cells, as well as transformation, culture, amplification, screening, and product generation and purification, are well known in the art.

It can be demonstrated that bacterial cells may be used as host cells and are suitable for expressing recombinant Fab or other embodiments of the present disclosure. However, because proteins expressed in bacterial cells tend to be unfolded or misfolded or non-glycosylated, any recombinant Fab generated in the bacterial cells must be screened to retain an antigen-binding ability. If the molecule expressed in the bacterial cell is generated in a properly folded form, the bacterial cell is a desired host, or in an alternative embodiment, the molecule may be expressed in the bacterial host and subsequently refolded. For example, various strains of Escherichia coli used for expression are well-known host cells in the field of biotechnology. Various strains of Bacillus subtilis, Streptomyces, other Bacillus, and the like can also be used in this method.

If required, yeast cell strains and cells of insects known to those skilled in the art, for example, drosophila, lepidopteran insects, and viral expression systems, can also be used as host cells.

In some embodiments, the nucleic acid is inserted into a cell genome and can be stably expressed.

The insertion may be conducted using the vector, or the nucleic acid may be transferred directly into cells without being inserted into the vector (via a lipid-mediated transfection technique).

The present disclosure further relates to a method for producing an antibody or an antigen-binding fragment thereof, including:
culturing the host cell under suitable culture conditions; and
recovering an antibody or antigen-binding fragment thereof thus produced from the culture medium or the cultured cells.

A culture method in the present disclosure is typically a serum-free culture method, and cells are typically cultured using a serum-free suspension. Similarly, once the antibody in the present disclosure has been generated, the antibody can be purified from cell culture contents based on a standard procedure in the art, and the standard procedure includes ammonium sulfate precipitation, affinity columns, column chromatography, gel electrophoresis, and the like. Such technology is within the technical scope of the art and is not limited to the present disclosure. Another antibody expression method may be the expression in animals (in particular transgenic animals or nude mice). This involves the use of the expression system of the animal casein promoter. When the animal casein promoter is transgenically incorporated into mammals, female animals can produce the desired recombinant protein in their milk. The medium with the secreted antibody may be purified via a conventional technique. For example, an A or G Sepharose FF column with adjusted buffer is used for purification. A non-specifically bound component is washed away. The bound antibody is then eluted using a pH gradient method, and the antibody fragment is detected via SDS-PAGE and collected. The antibody may be filtered and concentrated by a conventional method. Asoluble mixture and a multimer can also be removed by conventional methods, such as molecular sieving and ion exchange. The resulting product should be frozen immediately, such as at -70°C, or lyophilized.

The present disclosure further provides a conjugate, including the antibody or antigen-binding fragment thereof, and a conjugate moiety, where the conjugate moiety is selected from one or more of a radionuclide, a pharmaceutical, a toxin, a cytokine, a cytokine receptor fragment, an enzyme, fluorescein, and biotin.

The present disclosure further provides a composition or a combination product, including one or more of the antibody or antigen-binding fragment thereof (particularly, one or more of a to f, one or more of A to F, or one or more of B', C' and D'); and/or the conjugate.

In some embodiments, the composition further includes one or more of a pharmaceutically acceptable excipient, diluent, or vector.

The term "pharmaceutically acceptable excipient, diluent or vector" refers to an excipient, a diluent or a vector that is pharmaceutically and/or physiologically compatible with a subject and an active ingredient, and is well known in the art, including but not limited to: a pH regulator, a surfactant, an adjuvant, and an ionic strength enhancer. For example, the pH regulator includes, but is not limited to, a phosphate buffer; the surfactant includes, but is not limited to, a cationic, anionic or nonionic surfactant such as Tween-80; and the ionic strength enhancer includes, but is not limited to, sodium chloride.

Another aspect of the present disclosure further relates to the use of the antibody or antigen-binding fragment thereof, and/or the conjugate in preparation of a medicament, where the medicament is for treating one or more of tumors, asthma, irritable bowel disease, transplant rejection, autoimmune diabetes, graft-versus-host disease, experimental autoimmune encephalomyelitis, and atopic dermatitis.

In some embodiments, the tumor is selected from a hematological tumor, pancreatic cancer, bladder cancer, colorectal cancer, breast cancer, prostate cancer, kidney cancer, hepatocellular carcinoma, lung cancer, ovarian cancer, cervical cancer, gastric cancer, esophageal cancer, head and neck cancer, melanoma, neuroendocrine cancer, CNS cancer, brain tumor, bone cancer, and soft tissue sarcoma.

The embodiments of the present disclosure are described in detail below with reference to examples. It should be understood that these examples are used only to illustrate the present disclosure and are not intended to limit the scope of the present disclosure. In the following examples, for a test method for which a specific condition is not specified, it is preferable to refer to the guidance provided in the present disclosure, a test manual or a conventional condition in the art, another test method known in the art, or a condition recommended by a manufacturer.

In the following specific examples, a measurement parameter related to an ingredient may have a slight deviation within a weighing accuracy range unless otherwise specified. For temperature and time parameters, acceptable deviations caused by instrument test accuracy or operational accuracy are allowed.

### Example 1 Preparation, Screening and Subcloning of Murine Hybridoma Antibodies

(1) Mouse Immunization and Hybridoma Cell Fusion. OX40-ECD-mFc fusion protein was used as an antigen, and fully emulsified with an equal volume of complete Freund's adjuvant (Sigma, Cat. No.: F5581), then 6-8 week-old Balb/c mice (Zhaoyan (Suzhou) New Drug Research Center Co., Ltd.) were subcutaneously immunized. The antigen immunization dose was 20 µg/mouse. Subsequently, the mice were immunized subcutaneously three times every 2 weeks with the same dose of antigen fully emulsified in incomplete Freund's adjuvant (Sigma, Cat. No.: F5506). After three immunizations, serum titers of the mice were measured, and the mice were booster immunized intraperitoneally 3 days before the fusion. Using PEG Hybri-Max (Sigma, Cat. No.: 7181) as the fusion agent, spleen cells of the mice and SP2/0 cells were mixed at a ratio of 4:1. The fused cells were added to a 96-well plate (1×10⁵ cells/well), and each well contained 0.1 mL of 1X HAT medium (Invitrogen, Cat. No.: 21060-017). On day 3, 0.1 mL of HT medium (Invitrogen, Cat. No.: 11067-030) medium was added, and on day 7, the medium was pipetted out of the 96-well plate, and 0.2 mL of fresh HT medium was added. On day 9, a supernatant was collected for ELISA and FACS assays.
(2) ELISA Screening for Antibodies Binding to OX40-ECD. 96-well ELISA plate (Corning, Cat. No.: 9018) was inoculated with OX40-ECD-hFc, left at 4°C overnight, and washed 3 times with wash buffer (PBS+0.05% Tween 20), then blocking buffer (PBS+1% BSA (Sigma, Cat. No.: V90093)) was added, and the 96-well ELISA plate was incubated for 1 hour and washed 3 times; hybridoma supernatant was added, the 96-well ELISA plate was incubated for 1 hour and washed 3 times; and 100 µL of goat anti-mouse IgG secondary antibody (Thermo, Cat. No.: 31432) diluted at 1:10000 was added to each well, and the 96-well ELISA plate was incubated at room temperature for 1 hour and washed 3 times. 100 µL of TMB (Biosubstrate Technologies, Cat. No.: ES-002) was added to each well and left for 3 minutes for color development, then a stop solution (2N H₂SO₄) was added at a dose of 100 µL/well to terminate the reaction, and the OD450 signal of each sample was measured with a Tecan Spark microplate reader.
(3) FACS Screening for Antibodies Binding to OX40-ECD. 50 µL of the hybridoma supernatant tested positive in the ELISA tests was pipetted and mixed with 50 µL of 293T-OX40 cells (1×10⁵ cells/well), and the mixture was added to a 96-well plate with a U-shaped bottom and incubated for 1 hour. After the mixture was centrifuged and washed twice with FACS buffer (PBS+3% FCS), PE-labeled goat anti-mouse secondary antibody (Biolegend, Cat. No.: 405307) diluted at 1:400 was added, the mixture was incubated for 30 minutes, and washed with FACS buffer, and then PE signal of 293T-OX40 cells was detected using a BD C6 flow cytometer.
(4) The inhibitory effect of the hybridoma antibody on OX40L activation of the OX40 signaling pathway was tested using a reporter gene assay. 50 µL of hybridoma supernatant or purified hybridoma antibody that was positive in the ELISA test was taken and mixed with 25 µL of Jurkat-OX40 cells (4 × 10⁴ cells/well), a resulting mixture was added to a 96-well half-area flat-bottom plate, and then 25 µL of 1 µg/mL OX40L was added, mixed well, and then incubated in an incubator for 4 hours at 37°C. 25 µL of pre-heated Bright-Glo solution (Promega, Cat. No.: E2620) was added to each well, and left at room temperature in the dark for 3 minutes, and the luminescence signal value of each sample was measured by using a microplate reader (Tecan Spark). The Jurkat-OX40 cells used in this experiment were integrated with the NF-κB-luciferase gene.
(5) Subcloning of Hybridoma. Hybridoma having strong binding activity and capable of blocking the binding of OX40 to OX40L was subcloned using the limiting dilution method, and then the binding affinity of hybridoma antibody to OX40 was analyzed via ELISA and FACS, and the hybridoma monoclonal antibodies blocking the OX40L/OX40 signaling pathway were tested and screened using the reporter gene assay.

### Example 2 Testing of Antagonistic OX40 Monoclonal Hybridoma Antibodies

The positive monoclonal hybridoma cells were added to 50 mL of serum-free medium (Invitrogen, Cat. No.: 12045-076) and the serum-free medium was cultured for 9 days, and then centrifuged to collect the supernatant. The monoclonal antibody was purified using Protein A affinity chromatography. The purified antibody samples were liquid exchanged and concentrated in ultrafiltration centrifuge tubes (Millipore, Cat. No.: ACS500024). The protein concentration was determined using the BCA method, and limulus reagent (Xiamen Bioendo Technology Co., Ltd.) was used to detect the endotoxin content of the antibody samples.

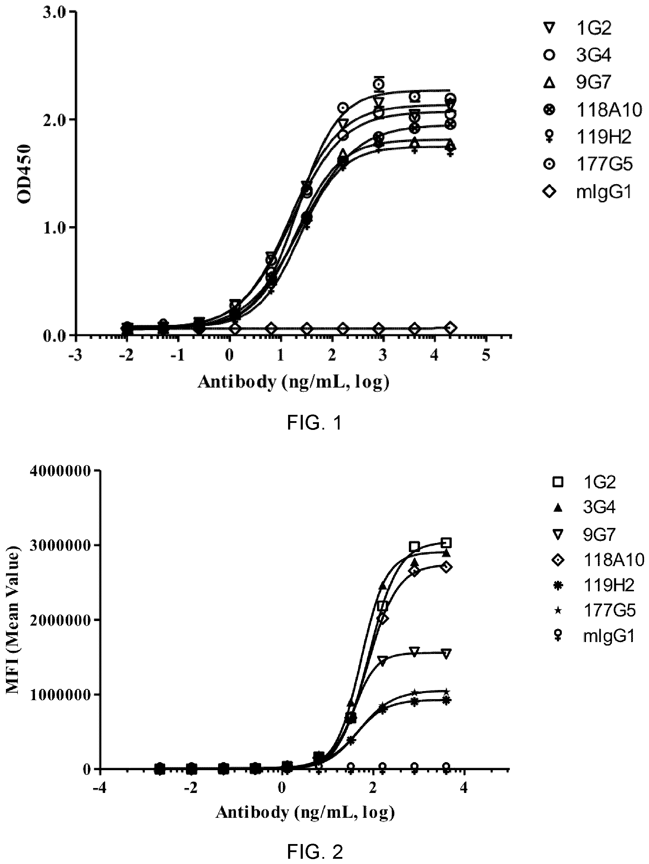

The activity of the purified antibody sample to bind OX40 and block the binding of OX40 to OX40L was tested via ELISA and FACS. Specific methods are described in steps (2), (3) and (4) in Example 1. 6 monoclonal OX40 hybridoma antibodies 1G2, 3G4, 9G7, 177G5, 118A10 and 119H2 were tested. Test results are shown in Table 1 to Table 3 and Figures 1 to 3.

**Table 1 ELSIAAssay of EC50 of OX40 Hybridoma Antibodies Binding to OX40**

| | 1G2 | 3G4 | 9G7 | 177G5 | 118A10 | 119H2 |
|---|---|---|---|---|---|---|
| EC50 (ng/mL) | 15.68 | 16.28 | 19.78 | 21.49 | 24.89 | 23.02 |

**Table 2 FACS Assay of EC50 of OX40 Hybridoma Antibodies Binding to 293T-OX40 Cells**

| | 1G2 | 3G4 | 9G7 | 177G5 | 118A10 | 119H2 |
|---|---|---|---|---|---|---|
| EC50 (ng/mL) | 80.53 | 53.71 | 36.90 | 49.90 | 73.52 | 41.26 |

**Table 3 Reporter Gene Assay of IC50 of OX40 Hybridoma Antibodies Blocking Activation of OX40L Induced Signal**

| | 1G2 | 3G4 | 9G7 | 177G5 | 118A10 | 119H2 |
|---|---|---|---|---|---|---|
| IC50 (ng/mL) | 117.3 | 36.06 | -36.14 | 253.0 | 198.0 | 178.6 |

The results show that murine OX40 hybridoma antibodies 1G3, 3G4, 9G7, 117G5, 118A10 and 119H2 have strong binding activity to human OX40 and can block the interaction between human OX40 and its ligand.

### Example 3 Gene Cloning of Variable Regions of Antagonistic OX40 Antibodies

Gene cloning and sequence analyses of variable regions of the 6 monoclonal hybridoma antibodies 1G2, 3G4, 9G7, 177G5, 118A10 and 119H2 were conducted in the present disclosure. A specific method is as follows: The OX40 monoclonal hybridoma cells were lysed with TRIzon (Cwbiotech, Cat. No.: CW0580), and the total RNA of the hybridoma cells was extracted. Hybridoma cell RNA was reverse transcribed into cDNA by using HiFi Script cDNA Synthesis Kit (Cwbiotech, Cat No: CW2569). The cDNA was used as a template, and degenerate primers were used to amplify the heavy chain and light chain variable region genes of the antibody in a PCR method (Kettleborough et al. (1993) Eur. J Immunology 23: 206-211; Strebe et al. (2010) Antibody Engineering 1:3-14). After the product of the PCR amplification was ligated to T/A vector, DH5α competent cells were transformed, inoculated and cultured overnight at 37°C. Monoclonal was picked from the culture plate and cultured for amplification, plasmids were extracted, and the gene sequence of the antibody was identified. Based on the gene sequence of the antibody, CDRs and framework regions of the antibody were analyzed. The numbers of the gene sequences and amino acid sequences of the variable regions of the heavy and light chains of some antibodies are shown in Table 4 below.

**Table 4 Number of Amino Acid Sequences of Variable Regions of Heavy and Light Chains of Antibodies**

| SEQ ID NO | Description | |
|---|---|---|
| | Antibody | Sequence description |
| 1 | 1G2 | Amino acid sequence of a heavy chain variable region |
| 2 | 1G2 | Amino acid sequence of a light chain variable region |
| 3 | 3G4 | Amino acid sequence of a heavy chain variable region |
| 4 | 3G4 | Amino acid sequence of a light chain variable region |
| 5 | 9G7 | Amino acid sequence of a heavy chain variable region |
| 6 | 9G7 | Amino acid sequence of a light chain variable region |
| 7 | 177G5 | Amino acid sequence of a heavy chain variable region |
| 8 | 177G5 | Amino acid sequence of a light chain variable region |
| 9 | 118A10 | Amino acid sequence of a heavy chain variable region |
| 10 | 118A10 | Amino acid sequence of a light chain variable region |
| 11 | 119H2 | Amino acid sequence of a heavy chain variable region |
| 12 | 119H2 | Amino acid sequence of a light chain variable region |
| 13 | 1G2 | Amino acid sequence of a heavy chain CDR1 region |
| 14 | 1G2 | Amino acid sequence of a heavy chain CDR2 region |
| 15 | 1G2 | Amino acid sequence of a heavy chain CDR3 region |
| 16 | 1G2 | Amino acid sequence of a light chain CDR1 region |
| 17 | 1G2 | Amino acid sequence of a light chain CDR2 region |
| 18 | 1G2 | Amino acid sequence of a light chain CDR3 region |
| 19 | 3G4 | Amino acid sequence of a heavy chain CDR1 region |
| 20 | 3G4 | Amino acid sequence of a heavy chain CDR2 region |
| 21 | 3G4 | Amino acid sequence of a heavy chain CDR3 region |
| 22 | 3G4 | Amino acid sequence of a light chain CDR1 region |
| 23 | 3G4 | Amino acid sequence of a light chain CDR2 region |
| 24 | 3G4 | Amino acid sequence of a light chain CDR3 region |
| 25 | 9G7 | Amino acid sequence of a heavy chain CDR1 region |
| 26 | 9G7 | Amino acid sequence of a heavy chain CDR2 region |
| 27 | 9G7 | Amino acid sequence of a heavy chain CDR3 region |
| 28 | 9G7 | Amino acid sequence of a light chain CDR1 region |
| 29 | 9G7 | Amino acid sequence of a light chain CDR2 region |
| 30 | 9G7 | Amino acid sequence of a light chain CDR3 region |
| 31 | 177G5 | Amino acid sequence of a heavy chain CDR1 region |
| 32 | 177G5 | Amino acid sequence of a heavy chain CDR2 region |
| 33 | 177G5 | Amino acid sequence of a heavy chain CDR3 region |
| 34 | 177G5 | Amino acid sequence of a light chain CDR1 region |
| 35 | 177G5 | Amino acid sequence of a light chain CDR2 region |
| 36 | 177G5 | Amino acid sequence of a light chain CDR3 region |
| 37 | 118A10 | Amino acid sequence of a heavy chain CDR1 region |
| 38 | 118A10 | Amino acid sequence of a heavy chain CDR2 region |
| 39 | 118A10 | Amino acid sequence of a heavy chain CDR3 region |
| 40 | 118A10 | Amino acid sequence of a light chain CDR1 region |
| 41 | 118A10 | Amino acid sequence of a light chain CDR2 region |
| 42 | 118A10 | Amino acid sequence of a light chain CDR3 region |
| 43 | 119H2 | Amino acid sequence of a heavy chain CDR1 region |
| 44 | 119H2 | Amino acid sequence of a heavy chain CDR2 region |
| 45 | 119H2 | Amino acid sequence of a heavy chain CDR3 region |
| 46 | 119H2 | Amino acid sequence of a light chain CDR1 region |
| 47 | 119H2 | Amino acid sequence of a light chain CDR2 region |
| 48 | 119H2 | Amino acid sequence of a light chain CDR3 region |

### Example 4 Preparation and Testing of Antagonistic OX40 Chimeric Antibodies Ch1G2, Ch3G4, Ch9G7, Ch177G5, Ch118A10 and Ch119H2

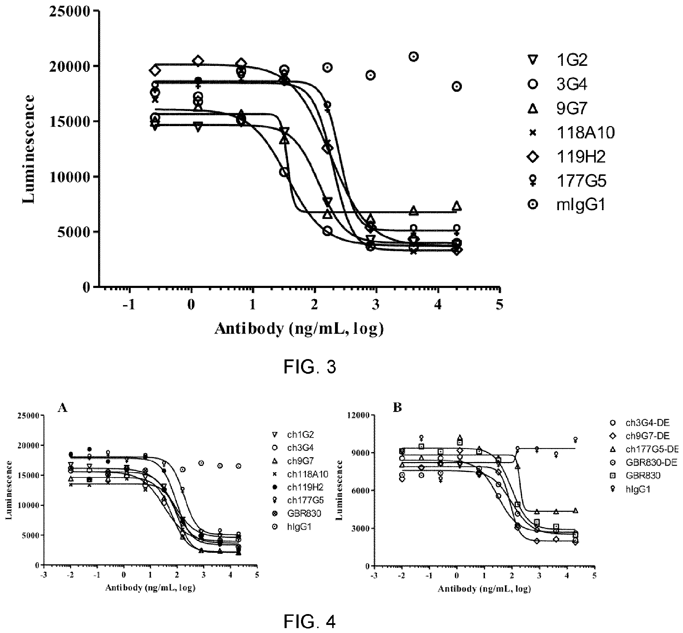

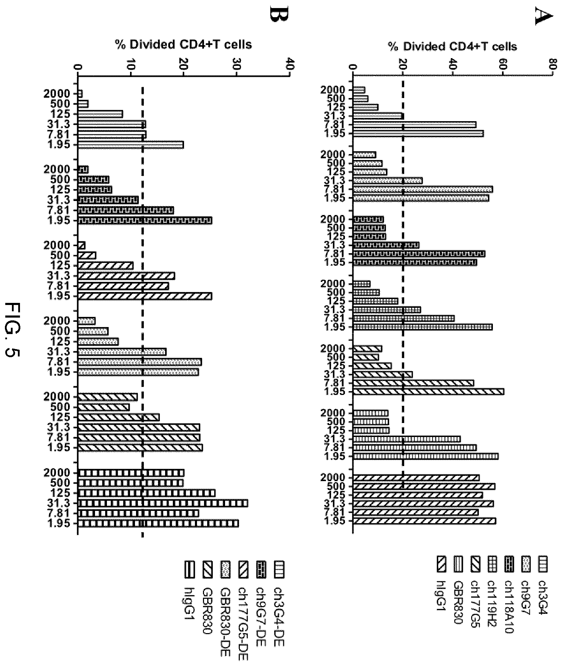

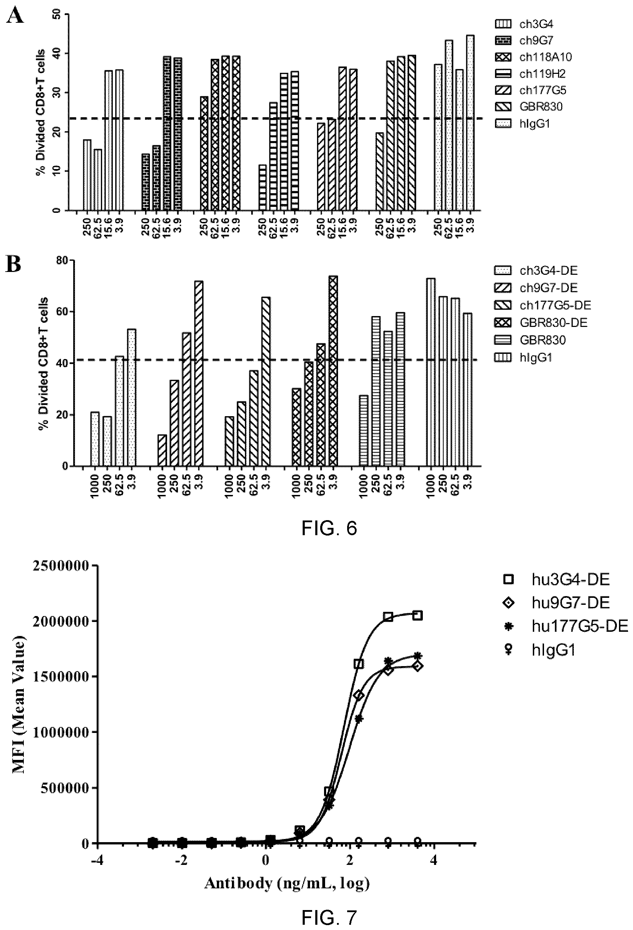

The murine Fc of hybridoma antibodies 1G2, 3G4, 9G7, 177G5, 118A10 and 119H2 were replaced with human Fc, to create human IgG1-based chimeric antibodies, and the constant regions Ser239, Ala330 and Ile332 of the antibodies were mutated to Asp, Leu, and Glu (namely, DLE mutations), respectively. Various tests were performed on these antibodies, including a reporter gene assay for the blocking effect of OX40 chimeric antibody on OX40L functions, and a MLR response assay for the inhibitory effect of OX40 chimeric antibody on CD4+ T cell proliferation, and its inhibitory effect on CEF-induced CD8+ memory T cell proliferation. The results are shown in Figure 4, Figure 5 and Figure 6 (GBR830 is a positive control antibody from Glenmark). The assay method is as follows:
(1) Testing the Blocking Effect of OX40 Chimeric Antibodies on OX40L Function Using the Reporter Gene Assay: The specific method is shown in step (4) in Example 1, and the results are shown in Figure 4.
(2) Testing the Inhibitory Effect of OX40 Chimeric Antibodies on CD4+ T Cell Proliferation using the MLR response assay: Cryopreserved PBMCs (Shanghai OriBiotech Co., Ltd. and Milestone (Shanghai) Biotechnology Co., Ltd.) were resuspended, added to pre-heated RPMI1640 complete medium, and mixed well, the resulting mixture was centrifuged at 1500 rpm for 5 minutes, complete medium containing mitomycin C (final concentration: 50 µg/mL) was added to resuspend the cells, the resulting mixture was put in 5% CO₂ incubator for incubation at 37°C for 2 hours, the cells were washed 3 times with the complete medium of 10 times volume and centrifuged, and then the cells were resuspended with the complete medium for counting and later use. Cryopreserved PBMCs of another donor were thawed, added to the pre-heated complete medium, mixed well, and centrifuged at 1500 rpm for 5 minutes, a resulting mixture was labeled with CFSE cell proliferation dye, a pre-chilled complete medium was added to quench free dye, the cells were washed 3 times, and the PBMCs were resuspended in the complete medium and counted. 100 µL of CFSE-labeled PBMC cells (2×10⁵/well) were added to a 96-well plate, 50 µL of diluted OX40 antibodies at different concentrations (final concentration: 2 µg/mL, 4-fold serial dilution) were added to the corresponding cell wells, 50 µL of mitomycin C-treated PBMC cells (6.5×10⁴/well) were added to the corresponding wells, and after the cells were mixed well, the 96-well cell culture plate was put in 5% CO₂ incubator for incubation at 37°C for 7 days, and the cells were collected after centrifugation. The cells were resuspended with a flow cytometry buffer (PBS+3% FCS), fluorescence-labeled anti-human CD4 antibodies and cell viability dye were added, a resulting mixture was incubated at 4°C for 1 hour, the cells were washed twice with the flow cytometry buffer, and the proportion of divided CD4+ T cells (CD4+ CFSElow/dim) in the total effector CD4+ T cells (CD4+ CFSE+) was tested and analyzed by using a flow cytometer. The OX40 antibodies listed in the present disclosure can significantly inhibit the proliferation of effector CD4+ T cells in MLR experiments. The experiment results are shown in Figure 5.
(3) Testing of the Inhibitory effect of OX40 Chimeric Antibody on CD8+ T Cell Proliferation Induced by CEF. Cryopreserved PBMCs (Shanghai OriBiotech Co., Ltd. and Milestone (Shanghai) Biotechnology Co., Ltd.) were resuspended, added to pre-heated RPMI1640 complete medium, mixed well, and centrifuged by a centrifuge at 1500 rpm for 5 minutes, a resulting mixture was labeled with CFSE cell proliferation dye, a pre-chilled complete medium was added to quench free dye, the cells were washed 3 times, and the PBMCs were resuspended in the complete medium and counted. 100 µL of CFSE-labeled PBMC cells (2×10⁵/well) were added to a 96-well plate, 50 µL of diluted OX40 antibodies at different concentrations (final concentration: 4 µg/mL, 4-fold serial dilution) were added to the corresponding cell wells, 50 µL of CEF peptide (final concentration: 200 ng/mL) was also added to the corresponding wells and mixed well, then the 96-well cell culture plate was put in 5% CO₂ incubator for incubation at 37°C for 7 days, and the cells were collected after centrifugation. The cells were resuspended with a flow cytometry buffer (PBS+3% FCS), fluorescence-labelled anti-human CD8 antibodies and cell viability dye were added, a resulting mixture was incubated at 4°C for 1 hour, the cells were washed twice with the flow cytometry buffer, and the proportion of divided CD8+ T cells (CD8+ CFSElow/dim) in the total effector CD8+ T cells (CD8+ CFSE+) was tested and analyzed by using a flow cytometer. The OX40 antibodies listed in the present disclosure can all significantly inhibit proliferation of memory CD8+ T cells induced by CEF. The experiment results are shown in Figure 6.

The results show that OX40 chimeric antibodies can block the OX40/OX40 signaling pathway, and inhibit the proliferation of CD4+ T cells and the proliferation of CD8+ memory T cells induced by CEF.

### Example 5 Humanization of Antagonistic Murine OX40 Antibodies 3G4, 9G7 and 177G5

Based on the activity of each chimeric antibody, the hybridoma antibodies 3G4, 9G7 and 177G5 were humanized.

The complementarity-determining region grafting method was used to humanize the OX40 antibodies. First, the IMGT database was queried for human germline antibody (germline antibody) sequences with the maximum homology to the light chain and heavy chain variable regions of murine antibodies Germline IGKV1-33*01 was selected for humanization of the light chain variable region of the antibody 3G4, and IGHV1-3*01 was selected for humanization of the heavy chain variable region. Germline IGKV4-1*01 was selected for the humanization of the light chain variable region of the antibody 9G7, and IGHV3-48*01 was selected for the humanization of the heavy chain variable region. IGKV1-39*01 was selected for the humanization of the light chain of the antibody 177G5, and IGHV3-11*01 was selected for the humanization of the heavy chain. The CDR region of the murine antibody was retained, and the framework sequence of the murine antibody was replaced with the framework sequence of the human germ line antibody. Secondly, a structural model of the murine antibody was created, and different amino acid sites in the structural models of the human antibody and the corresponding murine antibody were compared one by one. If the spatial structure of the CDR region was not damaged or changed when a human amino acid sequence was used at a specific site in the framework, the human amino acid sequence was used at the specific site, or otherwise, the corresponding murine sequence (that is, reverse mutation to the murine sequence) was used at the site. According to structure simulation, some amino acids of the framework regions of humanized antibodies were back-mutated to the murine sequence.

Thr at the 28^{th} site of a humanized heavy chain of the antibody 3G4 was back-mutated to Arg, Met at the 48^{th} site was reverse mutated to Ile, Val at the 67^{th} site was back-mutated to Ala, Ile at the 69^{th} site was back-mutated to Leu, Arg at the 71^{st} site was back-mutated to Ser, Thr at the 73^{rd} site was back-mutated to Lys, and Ala at the 93^{rd} site was back-mutated to Ser. Ser at the 49^{th} site of a humanized heavy chain of antibody 9G7 was back-mutated to Ala. Ile at the 48^{th} site of a humanized light chain of the antibody 9G7 was back-mutated to Val. Ser at the 49^{th} site of a humanized heavy chain of antibody 177G5 was back-mutated to Ala. Tyr at the 79^{th} site was back-mutated to Asn. Ile at 48^{th} site of a humanized light chain of antibody 177G5 was back-mutated to Val, and Phe at the 71^{st} site was back-mutated to Tyr.

As shown in Table 5, the amino acid sequence numbers of the heavy chain and light chain variable regions of the humanized antibody hu3G4 are SEQ ID NO: 49 and SEQ ID NO: 50, respectively. The amino acid sequence numbers of the heavy chain and light chain variable regions of the humanized antibody hu9G7 are SEQ ID NO: 51 and SEQ ID NO: 52, respectively. The amino acid sequence numbers of the heavy chain and light chain variable regions of the humanized antibody hu177G5 are SEQ ID NO: 53 and SEQ ID NO: 54, respectively. The humanized antibodies 3G4, 9G7 and 177G5 were constructed as IgG1 isoforms, and the constant regions Ser239, Ala330 and Ile332 were mutated to Asp, Leu and Glu, respectively.

**Table 5 Amino Acid Sequence Numbers of Humanized Anti-OX40 Antibodies**

| SEQ ID NO. | Description | |
|---|---|---|
| | Humanized Antibody | Amino acid sequence |
| 49 | hu3G4 | Humanized heavy chain |
| 50 | hu3G4 | Humanized light chain |
| 51 | hu9G7 | Humanized heavy chain |
| 52 | hu9G7 | Humanized light chain |
| 53 | hu177G5 | Humanized heavy chain |
| 54 | hu177G5 | Humanized light chain |

Nucleic acid sequences encoding the light and heavy chains of humanized antibodies 3G4, 9G7 and 177G5 were synthesized and inserted into expression vector pcDNA3.1. 200 mL of 293 cells (cell density: 1×10⁶) were co-transfected with 0.1 mg of antibody light chain expression plasmid and 0.1 mg of antibody heavy chain expression plasmid, and cultured at 37°C for 6 days with shaking, and the supernatant was collected after centrifugation. Protein A was used to purify the humanized antibodies and the purified humanized antibodies were tested for activity.

### Example 6 Activity Testing of Antagonistic Humanized Anti-OX40 Antibodies

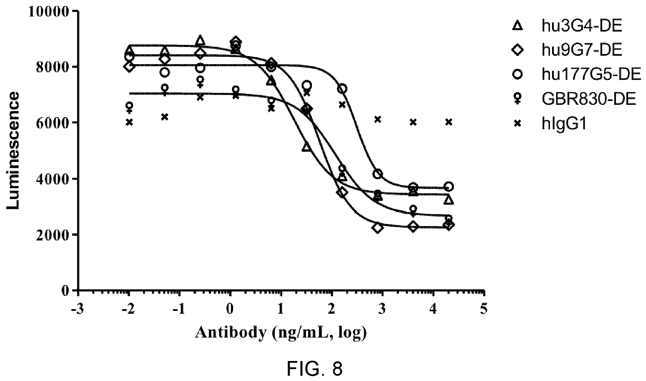

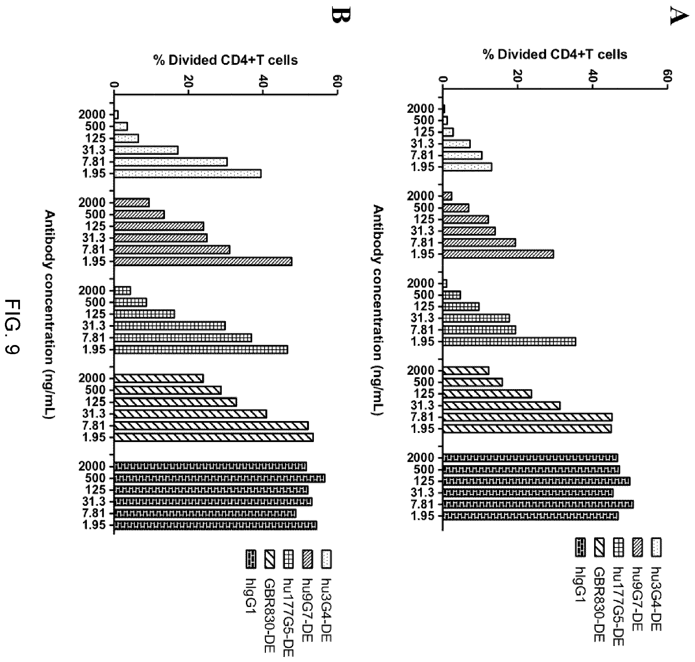

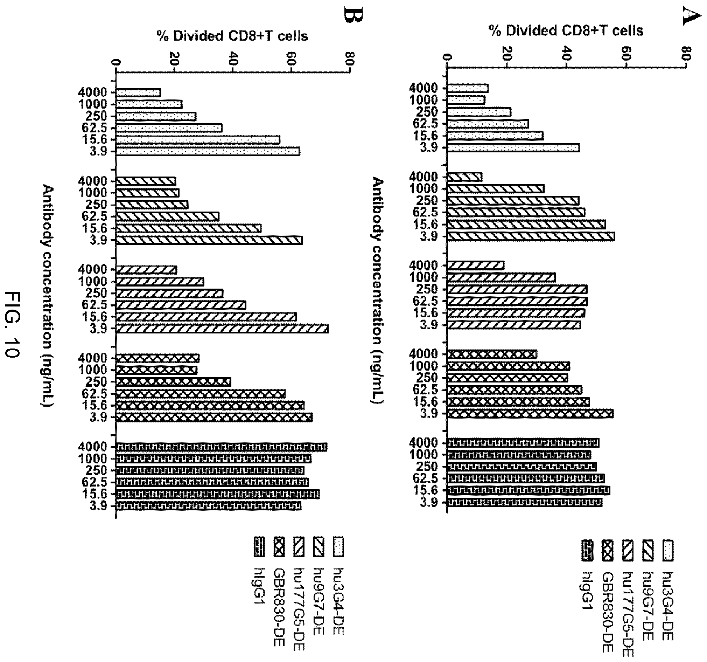

Purified humanized antibody samples were tested for their binding to OX40 using FACS and for their ability to block the OX40L/OX40 signaling pathway using a reporter gene assay. The specific methods are referenced from Example 1. The results are shown in Table 6, Table 7, Figure 7 and Figure 8. The effects of the humanized antibodies on the function of primary T cells were tested, including an MLR response test of the inhibitory effect of the OX40 humanized antibody on the proliferation of CD4+ T cells, and a test of the inhibitory effect of the OX40 humanized antibody on the proliferation of CD8+ T cells induced by CEF. The specific methods are referenced from Example 4, and the results are shown in Figure 9 and Figure 10.

**Table 6 FACS Assay of EC50 of OX40 Humanized Antibodies Binding to 293T-OX40 Cells**

| | hu3G4-DE | hu9G7-DE | hu177G5-DE |
|---|---|---|---|
| EC50 (ng/mL) | 71,32 | 62.29 | 95.96 |

**Table 7 Reporter Gene Assay of IC50 of OX40 Humanized Antibodies Blocking OX40L-Induced OX40 Signaling Pathway**

| | hu3G4-DE | hu9G7-DE | hu177G5-DE | GBR830-DE |
|---|---|---|---|---|
| IC50 (ng/mL) | 18.12 | 57.55 | 309.0 | 117.8 |

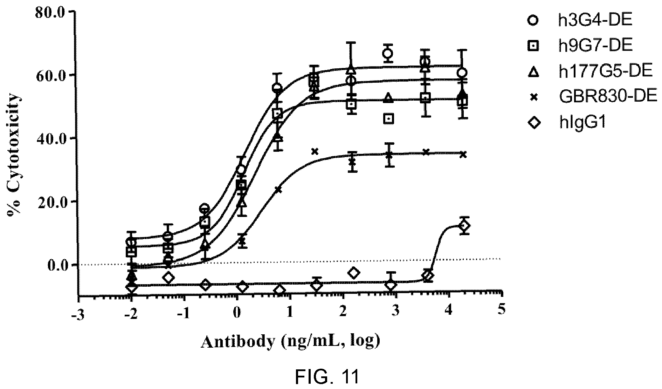

In addition, in the present disclosure, antibody-dependent cellular cytotoxicity (ADCC) was also tested for the antagonistic humanized anti-OX40 antibody. The antagonistic OX40 antibodies can further inhibit the inflammatory response by killing T cells with higher OX40 expression levels in the inflammatory response through ADCC activity. In the test, cryopreserved PBMCs (Shanghai OriBiotech Co., Ltd. and Milestone (Shanghai) Biotechnology Co., Ltd.) were resuspended, added to pre-heated RPMI1640 complete medium, and mixed well, a resulting mixture was centrifuged by a centrifuge at 1500 rpm for 5 minutes, the cells were resuspended with a complete medium, then the cells were added to a culture flask and put in 5% CO₂ incubator for incubation at 37°C for 18 hours, the PBMCs were collected after centrifugation, and then the cells were resuspended and counted. 25 µL of target cells Jurkat-OX40 (2×10⁴/well) were added to a 96-well plate, 25 µL of diluted OX40 antibodies at different concentrations (final concentration: 20 µg/mL, 5-fold serial dilution) were added to corresponding cell wells and mixed well, then the 96-well cell culture plate was put in 5% CO₂ incubator for incubation at 37°C for 30 minutes, 50 µL of PBMC cells (5×10⁵/well) were added to the corresponding wells, a separate control well was also set for each cell, and after the cells were mixed well, the 96-well cell culture plate was put in the incubator for incubation for 4 to 6 hours. The cells were centrifuged and the supernatant was collected, 50 µL of the supernatant was transferred to a new 96-well plate, 50 µL of LDH substrate chromogenic solution was added to each well, a resulting mixture was incubated for 20 to 30 minutes at room temperature, 50 µL of stop chromogenic solution was added, and a value of OD490 was measured with a microplate reader. The results show that the OX40 antibodies listed in the present disclosure all have significant ADCC activity and can induce the death of target cells. As shown in Table 8 and Figure 11, the antagonistic OX40 humanized antibody has strong ADCC activity against OX40-positive cells.

**Table 8 ADCC Activity of OX40 Humanized Antibodies**

| | hu3G4-DE | hu9G7-DE | hu177G5-DE | GBR830-DE |
|---|---|---|---|---|
| EC50 (ng/mL) | 1.522 | 1.426 | 2.364 | 3.054 |

The results show that the humanized antibodies hu3G4, hu9G7 and hu177G5 have high binding activity to OX40 and can block the OX40/OX40 signaling pathway, and notably, in the primary T cell function test, have a more significant inhibitory effect on the proliferation of CD4+ T cells and proliferation of CD8+ T cells induced by CEF compared to the positive control antibody GBR830, and also have strong ADCC activity for the OX40-positive cells.

In the examples, a mammalian cell expression system was used to prepare recombinant OX40 as an antigen for mouse immunization. Mouse spleen cells were fused with myeloma cells to obtain hybridoma cells. After multiple rounds of cloning and screening of a large number of hybridoma cells, several monoclonal hybridoma cell lines were obtained. These hybridoma cells can produce monoclonal antibodies with a high specific affinity for OX40, and these monoclonal antibodies can effectively block the binding of OX40 to OX40L, and inhibit the secretion of cytokines such as IL-2 and INF-γ in the response of mixed lymphocytes including memory T cells, and the proliferation of T cells. The genes encoding the light chain and heavy chain variable regions of the antibody were cloned by RT-PCR, and humanized antibodies were constructed using the complementarity-determining region grafting method. In vitro functional tests showed that these humanized OX40 antibodies can bind to OX40 protein with high specific affinity, efficiently block the binding of OX40L to OX40, and can inhibit secretion of cytokines such as IL-2 and INF-γ by T cells as well as T cell proliferation. The test results showed that the monoclonal antibodies or antigen-binding fragments thereof in the present disclosure, or conjugates containing the monoclonal antibodies or antigen-binding fragments thereof in the present disclosure have promising applications in the preparation of medicaments for blocking the binding of OX40L to OX40, medicaments for relieving OX40-mediated immune activation, medicaments for inhibiting T lymphocytes in inflammatory diseases, medicaments for reducing IL-2 and INF-γ expression in T lymphocytes, and medicaments for preventing and treating or adjuvantly treating chronic inflammations or autoimmune diseases.

The examples describe only several embodiments of the present disclosure. Although the descriptions are detailed, the descriptions should not be construed as a limitation on the scope of the invention. It should be noted that those of ordinary skills in the art could also make various variations and improvements without departing from the concept of the present disclosure. These variations and improvements all fall within the protection scope of the present disclosure. Therefore, the appended claims shall prevail over the protection scope of the present invention, and the description and drawings may be used to illustrate the content of the claims.

## Claims

1. An antibody or an antigen-binding fragment thereof capable of specifically recognizing OX40, comprising heavy chain complementarity-determining regions HCDR1, HCDR2, and HCDR3, and light chain complementarity-determining regions LCDR1, LCDR2, and LCDR3, wherein the amino acid sequences of HCDR1, HCDR2, HCDR3, LCDR1, LCDR2, and LCDR3 are selected from one of complementarity-determining region combinations designated as a to j:
| Combination name | HCDR1 | HCDR2 | HCDR3 | LCDR1 | LCDR2 | LCDR3 |
|---|---|---|---|---|---|---|
| a | SEQ ID NO: 13 | SEQ ID NO: 14 | SEQ ID NO: 15 | SEQ ID NO: 16 | SEQ ID NO: 17 | SEQ ID NO: 18 |
| b | SEQ ID NO: 19 | SEQ ID NO: 20 | SEQ ID NO: 21 | SEQ ID NO: 22 | SEQ ID NO: 23 | SEQ ID NO: 24 |
| c | SEQ ID NO: 25 | SEQ ID NO: 8 | SEQ ID NO: 9 | SEQ ID NO: 37 | SEQ ID NO: 38 | SEQ ID NO: 39 |
| d | SEQ ID NO: 10 | SEQ ID NO: 11 | SEQ ID NO: 12 | SEQ ID NO: 40 | SEQ ID NO: 41 | SEQ ID NO: 42 |
| e | SEQ ID NO: 13 | SEQ ID NO: 14 | SEQ ID NO: 15 | SEQ ID NO: 43 | SEQ ID NO: 44 | SEQ ID NO: 45 |
| f | SEQ ID NO: 16 | SEQ ID NO: 17 | SEQ ID NO: 18 | SEQ ID NO: 46 | SEQ ID NO: 47 | SEQ ID NO: 48 |

2. The antibody or the antigen-binding fragment thereof of claim 1, comprising a variable region, wherein a heavy chain variable region (HCVR) and a light chain variable region (LCVR) of the variable region have a sequence combination selected from one of variable region sequence combinations designated as A to F:
| Combination name | HCVR | LCVR |
|---|---|---|
| A | SEQ ID NO: 1 | SEQ ID NO: 2 |
| B | SEQ ID NO: 3 | SEQ ID NO: 4 |
| C | SEQ ID NO: 5 | SEQ ID NO: 6 |
| D | SEQ ID NO: 7 | SEQ ID NO: 8 |
| E | SEQ ID NO: 9 | SEQ ID NO: 10 |
| F | SEQ ID NO: 11 | SEQ ID NO: 12 |

3. The antibody or the antigen-binding fragment of claim 1, wherein the complementarity-determining region combination is selected from one of the complementarity-determining region combinations designated as b, c, and d, the antibody or the antigen-binding fragment thereof has a humanized variable region, wherein humanized heavy chain variable region (HCVR) and light chain variable region (LCVR) combinations of the respective complementarity-determining region combinations designated as b, c, and d are preferably designated as B', C' and D', respectively:
| Combination name | HCVR | LCVR |
|---|---|---|
| B' | SEQ ID NO: 49 | SEQ ID NO: 50 |
| C' | SEQ ID NO: 51 | SEQ ID NO: 52 |
| D' | SEQ ID NO: 53 | SEQ ID NO: 54 |

4. The antibody or the antigen-binding fragment thereof of any one of claims 1 to 3, wherein the antibody has a constant region, wherein a heavy chain constant region sequence is selected from any constant region sequence of IgG, IgA, IgM, IgE, and IgD; and a light chain constant region is a κ or λ chain.

5. The antibody or the antigen-binding fragment of claim 4, wherein a species origin of the heavy chain constant region and/or the light chain constant region is selected from rat, mouse, rabbit, cattle, horse, pig, sheep, dog, cat, camel, donkey, deer, marten, chicken, duck, goose, or human.

6. The antibody or the antigen-binding fragment of claim 5, wherein the heavy chain constant region is human IgG1, with Ser239, Ala330, and Ile332 mutated to Asp, Leu, and Glu, respectively.

7. The antibody or the antigen-binding fragment thereof of any one of claims 1 to 3, wherein the antigen-binding fragment is one of F(ab')₂, Fab, scFv, and a bispecific antibody.

8. An isolated nucleic acid encoding the antibody or the antigen-binding fragment thereof of any one of claims 1 to 7.

9. A vector comprising the nucleic acid of claim 8.

10. A host cell comprising the nucleic acid of claim 8 or the vector of claim 9.

11. A method for producing an antibody or an antigen-binding fragment thereof, comprising:
culturing the host cell of claim 10 under suitable culture conditions; and
recovering an antibody or antigen-binding fragment thereof thus produced from a culture medium or cultured cells.

12. A conjugate comprising the antibody or the antigen-binding fragment thereof of any one of claims 1 to 7 and a conjugate moiety, wherein the conjugate moiety is selected from one or more of a radionuclide, a pharmaceutical, a toxin, a cytokine, a cytokine receptor fragment, an enzyme, fluorescein, and biotin.

13. A composition or combination product, comprising one or more of the antibody or the antigen-binding fragment thereof of any one of claims 1 to 7; and/or the conjugate of claim 12.

14. The composition of claim 13, further comprising one or more of a pharmaceutically acceptable excipient, diluent, or vector.

15. Use of the antibody or the antigen-binding fragment thereof of any one of claims 1 to 7, and/or the conjugate of claim 12 in preparation of a medicament, wherein the medicament is for treating one or more of tumors, asthma, irritable bowel disease, transplant rejection, autoimmune diabetes, graft-versus-host disease, experimental autoimmune encephalomyelitis, and atopic dermatitis.
